# EUROPEAN PATENT SPECIFICATION

(11) **EP 4 656 590 B1**
(45) Date of publication and mention of the grant of the patent: **02.09.2026**
(21) Application number: 24178909.8
(22) Date of filing: 29.05.2024
(51) Int. Cl.: C01B 3/042, C01B 3/0015, C25B 15/08, C25B 1/04, C07C 51/00

(54) **WATER-EFFICIENT METHOD OF STORING HYDROGEN USING A BICARBONATE/FORMATE BASED REACTION SYSTEM**
WASSEREFFIZIENTES VERFAHREN ZUR SPEICHERUNG VON WASSERSTOFF UNTER VERWENDUNG EINES REAKTIONSSYSTEMS AUF BICARBONAT/FORMATBASIS
PROCÉDÉ DE STOCKAGE D'HYDROGÈNE À HAUT RENDEMENT DANS L'EAU UTILISANT UN SYSTÈME DE RÉACTION À BASE DE BICARBONATE/FORMATE

(43) Date of publication of application: 03.12.2025
(73) Proprietor: AKROS Energy GmbH, 18299 Rostock-Laage (DE)
(72) Inventor: Massa, Jonas, 18299 Rostock-Laage (DE); Turan, Volkan, 18299 Rostock-Laage (DE); Sponholz Peter, 18299 Rostock-Laage (DE)
(74) Representative: Hoffmann Eitle

(56) References cited:
- WO-A1-2024/105675
- CN-A- 113 278 995
- US-A1- 2022 177 399

## Description

### Field of the invention

The present invention relates to a method of storing hydrogen using a bicarbonate/formate-based aqueous reaction system and an apparatus configured for the same.

### Background art

Anthropogenic CO₂ emissions are a leading contributor to global warming. One way to reduce the reliance on CO₂-emitting fossil fuels is to transition towards green renewables such as wind and solar energy sources. Unlike the chemical energy storage of fossil fuels, solar and wind can only intermittently provide energy. A chemical storage system which buffers excesses and deficiencies in energy generated from solar and wind sources is therefore highly desired.

One candidate for chemical energy storage is hydrogen. Hydrogen can be generated from electrolysis of water using surplus electricity provided by wind and solar sources during peak times. The gas can be stored and the energy can be released on demand, e.g. in a hydrogen fuel cell. However, gaseous hydrogen is unsuitable for storage since it has a very low volumetric energy density and thus relies on liquefaction or compression in a high-pressure cylinder for storage. Both processes are highly energy intensive and require extensive safety measures.

It is therefore desirable to contain hydrogen in a chemical storage system for reversible storage and release. One proposed solution is a bicarbonate/formate-based aqueous reaction system. This system relies on the reduction of bicarbonate to formate salts or formic acid in order to store hydrogen, and the oxidation of formate to bicarbonate to release hydrogen. The system can be described according to the following exemplary reaction formulae:

KHCO₃ + H₂ -> HCOOK + H₂O Eqn. 1

HCOOK + H₂O -> H₂ + KHCO₃ Eqn. 2

However, in a method of storing hydrogen using the reaction system above, the generation/production of hydrogen requires extensive amounts of water making a method of storing hydrogen using the reaction system typically unsuitable for places with low water supplies, such as deserts. In particular, the reaction system requires the input of hydrogen, most of which is industrially generated by electrolysis ("green hydrogen") or steam cracking methane, which causes further CO₂ emissions, whereby large amounts of water are required in both cases. The present invention is therefore made with the motivation to provide a water efficient and low CO₂-emission method of storing hydrogen. WO2024/105675A1 relates to a continuous process for releasing hydrogen using a dehydrogenation reaction of a formate - bicarbonate cycle.

### Brief discussion of the present invention

In a first aspect, the present invention relates to a method of storing hydrogen using a bicarbonate/formate-based aqueous reaction system, wherein the method comprises:
(A) reducing aqueous bicarbonate using hydrogen to form formate and water,
(B) at least partially separating water from the aqueous reaction system to provide water and concentrated salt components comprising formate, and
(C) using the water provided in step (B) to form hydrogen for use in step (A) and/or to dissolve concentrated salt components comprising bicarbonate to provide aqueous bicarbonate for use in step (A).

In a preferred embodiment, the step (A) is preceded by dissolving concentrated salt components comprising bicarbonate to provide aqueous bicarbonate, preferably wherein the concentrated salt components comprising bicarbonate are a bicarbonate salt.

In a preferred embodiment, (i) the aqueous bicarbonate of step (A) is at a concentration of 1-10 M, preferably 4-6 M, and/or (ii) step (A) is carried out in the presence of a catalyst, preferably wherein the catalyst is a heterogeneous catalyst, and/or (iii) step (A) is carried out at a temperature of 20-100 °C and a hydrogen pressure of 1-100 bar.

In a preferred embodiment, the at least partial separation of water is carried out by at least one of vacuum distillation, atmospheric distillation, vacuum evaporation, evaporation, membrane separation, reverse osmosis, crystallization, extraction, electrochemical separation or precipitation.

In a preferred embodiment, the concentrated salt components comprising formate further comprise bicarbonate; preferably wherein the molar ratio of formate to bicarbonate is at least 1:1, more preferably at least 4:1, and even more preferably at least 9:1.

In a preferred embodiment, the concentrated salt components comprising formate are separated from the aqueous reaction system to provide a formate salt, optionally wherein a further formate salt separation step is carried out.

In a preferred embodiment, the concentrated salt components comprising formate further comprise bicarbonate and wherein these concentrated salt components comprising formate and bicarbonate are recycled for use in step (A).

In a preferred embodiment, the forming of hydrogen is carried out by any of photosynthesis, thermal decomposition of water or electrolysis, preferably at least one of direct electrolysis, alkaline electrolysis, high temperature electrolysis, high pressure electrolysis, proton-exchange membrane electrolysis, anion exchange electrolysis, and supercritical water electrolysis.

In a preferred embodiment, the method comprises:
(a) carrying out step (A) in a reduction reactor configured to execute the process of step (A),
(b) carrying out step (B) in at least one separating unit configured to execute the process of step (B),
(c) forming the hydrogen for use in step (A) using the water provided in step (B) in a hydrogen generating unit, and/or transferring the water provided in step (B) to the reduction reactor.

In a second aspect, the present invention relates to an apparatus configured for storing hydrogen using a bicarbonate/formate-based aqueous reaction system, wherein the apparatus comprises:
(alpha) a reduction reactor configured to reduce aqueous bicarbonate using hydrogen to form formate and water,
(beta) at least one separating unit configured to at least partially separate water from the aqueous reaction system to provide water and concentrated salt components comprising formate, wherein the separating unit is in communication with the reduction reactor, and
(gamma) a hydrogen generating unit in communication with the at least one separating unit and with the reduction reactor.

### Brief discussion of the figures

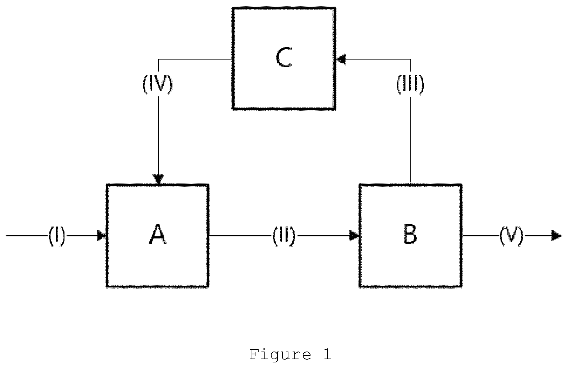

Figure 1 shows an exemplary embodiment of the present invention.

### Detailed discussion of the present invention

Hereafter, the present invention will be discussed in detail.

### Method of storing hydrogen

In a first aspect, the present invention relates to a method of storing hydrogen using a bicarbonate/formate-based aqueous reaction system, wherein the method comprises:
(A) reducing aqueous bicarbonate using hydrogen to form formate and water,
(B) at least partially separating water from the aqueous reaction system to provide water and concentrated salt components comprising formate, and
(C) using the water provided in step (B) to form hydrogen for use in step (A) and/or to dissolve concentrated salt components comprising bicarbonate to provide aqueous bicarbonate for use in step (A).

The presently claimed method uses a bicarbonate/formate-based aqueous reaction system for storing hydrogen. The bicarbonate/formate-based aqueous reaction system is an aqueous reaction system comprising bicarbonate salts. Typically, this will be in the form of an aqueous solution comprising bicarbonate and formate salts. Unless otherwise specified, the simpler expression "the reaction system" may also refer to the bicarbonate/formate-based aqueous reaction system. Reactions occurring in the reaction system are a 'hydrogen-storage' hydrogenation reaction of bicarbonate and a 'hydrogen-release' dehydrogenation reaction of formate. Typically, the bicarbonate/formate-based aqueous reaction system substantially consists of bicarbonate and formate salts dissolved in water. In some embodiments, carbonate salts may be further present, e.g. as a decomposition product of bicarbonate. In this embodiment, the bicarbonate/formate-based aqueous reaction system substantially consists of bicarbonate, formate and carbonate salts. The reaction system has thus the components formate salt (A₁HCOO), bicarbonate salt (A₂HCO₃) and optionally carbonate salt (A₃CO₃), wherein A₁, A₂ and A₃ are counterions, dissolved in water. Preferably, the reaction system comprises at least 75 wt% of the components A₁HCOO, A₂HCO₃, and optionally A₃CO₃, more preferably at least 80 wt%, more preferably at least 90 wt%, more preferably at least 95 wt%, more preferably at least 99 wt%, and even more preferably at least 99.9 wt%. The above mentioned wt% are relative to the total dry weight of the reaction system.

In a preferred embodiment, the bicarbonate/formate-based aqueous reaction system comprises substantially one type of counterion. Consequently, A₁ and A₂ and optionally A₃ are all the same. Typically, no further counterions are present other than those associated with the typical impurities of the salt. For instance, in one embodiment A₁, A₂ and (where present) A₃ being all the same, comprise at least 95 mol% of the counterions to the bicarbonate, formate, and (where present) carbonate in the aqueous reaction system, preferably at least 97 mol%, and even more preferably at least 99 mol%. In a particularly preferred embodiment, the counterions are K⁺-ions, meaning that A₁, A₂ and (where present) A₃ are all K⁺.

In the present context of the aforementioned reaction system, a "method of storing hydrogen using a bicarbonate/formate-based aqueous reaction system" refers to a method of storing hydrogen by reducing the bicarbonate using hydrogen to form formate and water according to exemplary chemical equation (1).

KHCO₃ + H₂ -> KHCOO + H₂O (1).

The bicarbonate (HCO₃⁻) is thus a hydrogen carrier precursor and the formate (HCOO⁻) is a hydrogen carrier. A method of releasing the hydrogen from the hydrogen carrier in the reverse direction of eqn. (1) is described elsewhere. The "method of storing hydrogen using a bicarbonate/formate-based aqueous reaction system" includes but is not exclusively limited to the aforementioned reaction system. For instance, the method may include the use of dry components such as bicarbonate and formate salts (e.g. for shipping to and from the place where the hydrogenation reaction takes place) and the use of gaseous components, such as hydrogen gas. The method of storing hydrogen using a bicarbonate/formate-based aqueous reaction system involves customary and common steps. These are, *inter alia,* providing a reaction vessel, such as a reactor suitable to carry out the methods; providing the reagents of the reaction system discussed below, such as bicarbonate salts, formate salts, water and hydrogen; providing water as an aqueous medium; setting appropriate temperature, pressure and reaction run times; and separating the products.

The presently claimed method comprises a step (A) of reducing aqueous bicarbonate using hydrogen to form formate and water. The chemical reaction taking place in step (A) is described by eqn. (1). As is known in the art, the reaction of eqn. (1) is reversible, and it will thus be appreciated that the position of the equilibrium may not entirely shift towards the products formate and water. Step (A) also does not preclude the occurrence of unwanted side-reactions. "Reducing aqueous bicarbonate using hydrogen" as used herein means contacting an aqueous solution and/or suspension of a bicarbonate salt with hydrogen gas at an appropriate temperature and pressure to allow the aforementioned reaction to occur. The aqueous solution and/or suspension of the bicarbonate salt is part of the aqueous reaction system described further above. Depending on the temperature and pressure, the water may be in the form of liquid water, vapour or steam.

The presently claimed method comprises a step (B) of at least partially separating water from the aqueous reaction system to provide water and concentrated salt components comprising formate. The term "concentrated salt components comprising formate" here refers to components substantially comprising salts including the formate formed in step (A). Typically, the concentrated salt components comprising formate will substantially consist of formate and bicarbonate salts whose molar ratio is reflective of the position of equilibrium in the reaction system, and optionally water. The precise composition of the concentrated salt components comprising formate in preferred embodiments is discussed in more detail below. In this specific context, "concentrated" is a relative term referring to the relatively high concentration of the salt components after the separation of water. Depending, *inter alia,* on the amount of water removed in step (B), the concentrated salt components comprising formate may be a concentrated aqueous solution, an aqueous suspension, or a solid. In a particularly preferred embodiment, the amount of water in moles separated in step (B) is at least the amount of water formed in step (A). In this embodiment, at least the amount of water formed in step (A) is provided. Unless otherwise specified, the "amount of X" as used herein refers to the amount of X in moles.

The presently claimed method comprises a step (C) of using the water provided in step (B) to form hydrogen for use in step (A) and/or to dissolve concentrated salt components comprising bicarbonate to provide aqueous bicarbonate for use in step (A). As will be understood, "for use in step (A)" in this context refers to a further instance of step (A), such as a second step (A) after a first step (A). This is in accordance with the claimed process having a cyclical nature.

In a preferred embodiment, the water provided in step (B) may be used to form hydrogen for use in step (A). In a particularly preferred embodiment, at least the amount of water formed in step (A) is provided in step (B) and used to form hydrogen for use in step (A). In this embodiment, the amount of hydrogen formed for use in step (A) is at least as high as the amount of water formed in step (A).

In another preferred embodiment, the water provided in step (B) is used to dissolve concentrated salt components comprising bicarbonate to provide aqueous bicarbonate for use in step (A). The term "concentrated salt components comprising bicarbonate" refers to components substantially comprising bicarbonate salts. These concentrated salt components comprise bicarbonate salts. In some embodiments, these concentrated salt components consist of bicarbonate salts and the typical impurities of the salts, such as the carbonate decomposition product. In other embodiments, the concentrated salt components comprising bicarbonate further comprise formate. In one embodiment further discussed below, the concentrated salt components comprising formate and bicarbonate may be recycled for use in step (A). In the context of "concentrated salt components comprising bicarbonate", "concentrated" is a relative term referring to the relatively high concentration of the salt components before dissolving using the water provided in step (B). The concentrated salt components comprising bicarbonate may be solid, such as solid bicarbonate salt, in an aqueous suspension or in an aqueous solution. In the context of the latter two cases, 'dissolving' as used in step (C) may also refer to diluting.

In a preferred embodiment, the water provided in step (B) is used to form hydrogen for use in step (A) and to dissolve concentrated salt components comprising bicarbonate to provide aqueous bicarbonate for use in step (A).

Through the above disclosed means, the present inventors have provided a water-efficient method of storing hydrogen using a typically water-intensive method of forming and storing hydrogen. The presently claimed method recovers water for generating hydrogen for storage and/or for dissolving salts of the aqueous reaction system. In particular, the water formed by the reduction of aqueous bicarbonate can be used for generating hydrogen in a stochiometric equivalent manner. This means that the amount of hydrogen thus generated is (substantially) the same as the amount of hydrogen used for reducing aqueous bicarbonate in the first instance of step (A). The hydrogen thus generated can be used for reducing aqueous bicarbonate in a second instance of step (A), thereby forming water in (substantially) the same amount as during the first instance of step (A). Through this process, the water cycle can be controlled and kept independent of the salt cycle. The presently disclosed method of storing hydrogen can thus be dynamically adjusted to the local relative water and energy costs. The hydrogen generation can also be kept independent and free of the need for external water or fossil fuels. This means that the above disclosed method is highly suitable in places where water supplies are low but green energy can readily be provided by means of wind and solar, such as deserts.

In a particularly preferred embodiment, the amount of water provided in step (B) is higher than the amount of water formed in step (A); and used to form hydrogen for use in step (A) and to dissolve concentrated salt components comprising bicarbonate for use in step (A). In a preferred embodiment, the amount of hydrogen formed in step (C) is sufficient for the aqueous bicarbonate reduction of step (A). In this embodiment, the hydrogen generation is completely independent of external water and fossil fuel supplies.

In one embodiment, substantially all water is separated from the aqueous reaction system in step (B) and consequently used to form hydrogen for use in step (A) and to dissolve concentrated salt components comprising bicarbonate in step (A). In this embodiment, substantially dry concentrated salt components comprising bicarbonate, e.g. a bicarbonate salt, can be provided and substantially dry concentrated salt components comprising formate, e.g. a formate salt, can be obtained, and a continuous water cycle is used for generating hydrogen and to solubilise the reaction system. In this embodiment, the entire method of storing hydrogen is substantially independent of external water and fossil fuel supplies. The only water losses in this embodiment which would have to be recovered by external supplies would be due to leaks, evaporation, and the like.

In one embodiment, the amount of water provided in step (B) is at least 15 mol%, relative to the total amount of water in the aqueous reaction system, preferably at least 25 mol%, preferably at least 30 mol%, preferably at least 40 mol%, preferably at least 50 mol%, preferably at least 60 mol%, preferably at least 70 mol%, preferably at least 80 mol%, preferably at least 90 mol%, preferably at least 95 mol%, and even more preferably at least 98 mol%.

In a preferred embodiment, the step (A) is preceeded by dissolving concentrated salt components comprising bicarbonate to provide aqueous bicarbonate. In one embodiment, the concentrated salt components comprising bicarbonate are a bicarbonate salt.

The term "concentrated salt components comprising bicarbonate" has the same meaning as the discussed above.

In the presently discussed embodiment, the dissolving of concentrated salt components comprising bicarbonate to provide aqueous bicarbonate may be part of the step (C) as discussed above but is not limited thereto. It may also include dissolving the salt components using externally provided water, i.e. water not provided in step (B), e.g. during the initial start-up of the process before any water is formed in step (A).

In a preferred embodiment, the aqueous bicarbonate of step (A) is at a concentration of 1-10 M, preferably 4-6 M. This concentration refers to the initial concentration of aqueous bicarbonate in the aqueous reaction system. In one embodiment, at the same time, the concentration of formate is zero, e.g. during the initial start-up. In other embodiments, such as the bicarbonate recycling discussed below, formate may be present.

In a preferred embodiment, the step (A) is carried out in the presence of a catalyst. Preferably, this catalyst is a heterogenous catalyst. The catalyst may not be particularly limited. Exemplary catalysts include Pd-based catalysts, such as Pd₄S, Pd, Pd-Au, Pd@Ag, Pd-Cu₂O, PdS, Pd/C, Pd/Al₂O₃. In a preferred embodiment, the catalyst is a Pd/C catalyst or a Pd/TiO2-based catalyst.

In a preferred embodiment, the step (A) is carried out at a temperature of 20-100 °C, and a hydrogen pressure of 1-100 bar. In one embodiment, the reduction of bicarbonate in step (A) takes place at a temperature of 20-100 °C, preferably 30-90 °C, preferably 40-80°C, preferably 50-70 °C, and even more preferably 55-65 °C. In one embodiment, the reduction of bicarbonate in step (A) takes place at a hydrogen pressure of 5-55 bar, preferably 10-50 bar, preferably 15-45 bar, preferably 20-40 bar, and even more preferably 25-35 bar. In one embodiment, the reduction of bicarbonate in step (A) takes place at a temperature of 20-100 °C and at a hydrogen pressure of 5-55 bar, preferably 30-90 °C and 10-50 bar, preferably 40-80°C and 15-45 bar, preferably 50-70 °C and 20-40 bar, and even more preferably 55-65 °C and 25-35 bar.

In a preferred embodiment, the at least partial separation of water is carried out by at least one of vacuum distillation, atmospheric distillation, vacuum evaporation, evaporation, membrane separation, reverse osmosis, crystallization, extraction, electrochemical separation (e.g., electrochemically via dialysis) or precipitation.

In a preferred embodiment, the concentrated salt components comprising formate further comprise bicarbonate. Preferably, the molar ratio of formate to bicarbonate is at least 1:1, more preferably at least 3:1, more preferably at least 6:1, and even more preferably at least 9:1.

The concentrated salt components comprising formate may further comprise bicarbonate if the position of the equilibrium in the reaction of step (A) is not fully shifted towards the side of the products formate and water. Depending on the position of the equilibrium, the molar ratio of bicarbonate to formate in the aqueous reaction system at the end of step (A) may differ. This, in turn, influences the molar ratio of formate to bicarbonate in the concentrated salt components comprising formate. The molar ratios given above reflect successively higher enrichments of formate.

In a preferred embodiment, the concentrated salt components comprising formate which further comprise bicarbonate are recycled for use in step (A). "Recycling for use in step (A)" refers to the provision of aqueous bicarbonate for use in step (A) from the concentrated salt components comprising formate and bicarbonate. This provided aqueous bicarbonate may also be referred to as "recycled bicarbonate". Recycling for use in step (A) may include further steps, such as dissolving and/or diluting in water, and/or separation steps as further discussed below. The water used for dissolving and/or diluting may be provided in step (B). In other words, this embodiment includes at least a first step (A), followed by a first step (B) to provide concentrated salt components comprising formate and bicarbonate, and recycling of the concentrated salt components comprising formate and bicarbonate for use in a second step (A). It may be the case that the concentrated salt components comprising formate and bicarbonate as provided in step (B) are recycled for use in step (A). However, it may also be the case that the concentrated salt components comprising formate and bicarbonate provided in step (B) are first separated (for example, the formate salts are separated from the bicarbonate salts in a formate separation step, as discussed further below) and then only the bicarbonate salts are recycled for use in step (A). The numbering of steps (first, second, etc) here is arbitrary and further recycling steps may be present. The number of recycling steps is not limited. The hydrogen formed in step (C) may of course be used in any instance of step (A).

In a preferred embodiment, the method typically includes adding further bicarbonate salt to the recycled bicarbonate before step (A). This further bicarbonate salt is preferably substantially pure bicarbonate salt. In this way, the molar ratio of formate to bicarbonate in the aqueous reaction system can be adjusted to enrich the aqueous reaction system with bicarbonate before step (A) and to remove formate from the aqueous reaction system at the end of step (B). The further added bicarbonate salt may be used to obtain the preferred initial concentration of bicarbonate discussed above. In this way, a salt cycle of bicarbonate and formate salts can be provided, wherein bicarbonate salts are added before step (A) and formate salts are removed at the end of step (B). Methods for removing the concentrated salt components comprising formate and optionally bicarbonate from the aqueous reaction system and optionally a further formate separation step are discussed below.

In a preferred embodiment, the presently claimed method is carried out in a cascade, wherein the method includes a first step (A), followed by a first step (B) to provide concentrated salt components comprising formate and bicarbonate, and recycling of the concentrated salt components comprising formate and bicarbonate for use in a second step (A), wherein the hydrogen used in the second step (A) is at a higher pressure than the hydrogen used in the first step (A). Through this method, the molar ratio of formate to bicarbonate in the concentrated salt components provided in step (B) can be successively increased.

In another preferred embodiment, the presently claimed method may also be carried out in a cascade in such a manner that the step (A) is performed multiple times, such as two or more times (for example in a plurality of reduction reactors arranged in series, as discussed further below) before a first step (B) is carried out, wherein the hydrogen used in the second step (A) is at a higher pressure than the hydrogen used in the first step (A). Through this method, the molar ratio of formate to bicarbonate in the concentrated salt components provided in step (B) can be successively increased.

In a preferred embodiment, the concentrated salt components comprising formate are separated from the aqueous reaction system to provide a formate salt. The method of separating the concentrated salt components comprising formate from the aqueous reaction system is not particularly limited. The method may include a further separation of water, as described in step (B), collection and removal of a precipitate formed during the step (B), separations based on density, differences in partial pressures, differences in solubilities, filtration, reverse osmosis, electrochemical separation and the like. The provided formate salt may be an aqueous solution, an aqueous dispersion or a solid. The provided formate salt may be substantially pure, i.e. comprising less than 10 mol% impurities, preferably less than 8 mol% impurities, preferably less than 5 mol% impurities, preferably less than 3 mol% impurities, and even more preferably less than 1 mol% impurities. Optionally, a further formate salt separation step may be carried out to increase the purity of formate. Such a separation step is not particularly limited and may include any separation steps used in the art. In one embodiment, the optional formate salt separation step comprises recrystallisation of formate.

In another preferred embodiment, the concentrated salt components comprising formate and bicarbonate are separated from the aqueous reaction system to provide a formate salt and a bicarbonate salt. Similar methods may be used to separate the concentrated salt components comprising formate and bicarbonate from the aqueous reaction system as discussed above for the concentrated salt components comprising formate. The provided formate salt and provided bicarbonate salt typically have the same form and content as the provided formate salt described in the preceding paragraph. In one embodiment, the formate salt and the bicarbonate salt are further separated from one another. This separation may be carried out using any known separation technique used in the art, such as density-based separation methods, electrochemical separation methods, thermal separation methods and/or crystallisation.

In a preferred embodiment, the forming of hydrogen in step (C) is carried out by any of photosynthesis, thermal decomposition of water, electrolysis, or a combination of any of these. Preferably, electrolysis is used. Any known water electrolysis method may be used such as at least one of direct electrolysis, alkaline electrolysis, high temperature electrolysis, high pressure electrolysis, proton-exchange membrane electrolysis, anion exchange electrolysis, and supercritical water electrolysis. Preferably, the energy required to power the electrolysis is provided by renewable energy means, such as wind and solar.

In a preferred embodiment, the method comprises:
(a) carrying out step (A) in a reduction reactor configured to execute the process of step (A),
(b) carrying out step (B) in at least one separating unit configured to execute the process of step (B),
(c) forming the hydrogen for use in step (A) using the water provided in step (B) in a hydrogen generating unit, and/or transferring the water provided in step (B) to the reduction reactor.

The reduction reactor, the at least one separating unit, and the hydrogen generating unit may be further characterised as described in the second aspect below. Transferring the water provided in step (B) to the reduction reactor is typically done by conduits providing a communication between the at least one separating unit and the reduction reactor. Likewise, transferring the water provided in step (B) to the hydrogen generating unit is typically done by (a) conduit(s) providing a communication between the at least one separating unit and the hydrogen generating unit. Transferring the hydrogen generated by the hydrogen generating unit in step (C) may be transferred to the reduction reactor by (a) conduit(s) providing a communication between the hydrogen generating unit and the reduction reactor. The method is further characterised in the second aspect below.

### Apparatus configured for storing hydrogen

In a second aspect, the present invention relates to an apparatus configured for storing hydrogen using a bicarbonate/formate-based aqueous reaction system, wherein the apparatus comprises:
(alpha) a reduction reactor configured to reduce aqueous bicarbonate using hydrogen to form formate and water,
(beta) at least one separating unit configured to at least partially separate water from the aqueous reaction system to provide water and concentrated salt components comprising formate, wherein the separating unit is in communication with the reduction reactor, and
(gamma) a hydrogen generating unit in communication with the separating and with the reduction reactor.

Where applicable, all definitions from the first aspect apply. Definitions from the second aspect also apply to the first aspect. This is especially the case for functional features of the apparatus described in the second aspect which apply to the last preferred embodiment of the preceding method.

Preferred embodiments of the preceding method are also discussed herein.

The reduction reactor is a reactor configured to reduce aqueous bicarbonate using hydrogen to formate and water. Accordingly, the reduction reactor is a vessel built to withstand elevated pressures and temperatures, such as the temperatures and pressures used in preferred embodiments of step (A) of the method of first aspect. Typical elements that the reduction reactor may include are a hydrogen inlet, an inlet and outlet for the aqueous reaction system, a through-flow section through which the aqueous reaction system flows, a heating element, an inert lining, a pressure sensor, a pressure release safety valve, a stirrer, a mounting for a fixed catalyst bed. The heating element may be arranged outside of the reactor, in particular outside of the through-flow section, for example at a conduit for supplying the aqueous reaction system into the reactor (i.e., into the through-flow section). In this way, complexity and costs of the reactor can be reduced since the reactor does not need a complex internal structure for supplying or transferring heat for hydrogenating the reaction system.

The presently claimed apparatus may include more than one reduction reactor. A plurality of reduction reactors may be present which are connected in parallel or in sequence. In one embodiment, a plurality of reduction reactions is present which are charged with increasing pressures of hydrogen. This embodiment is configured to carry out the cascade method described above.

The at least one separating unit is a separating unit configured to at least partially separate water from the aqueous reaction system to provide water and concentrated salt components comprising formate. The at least one separating unit is not particularly limited, and any separating units typically used in the art may be used so long as they are configured for the above process. One separating unit may be used, or a plurality of separating units may be used. These may be arranged in sequence or in parallel. For example, the at least one separating unit may be additionally configured to perform a formate separating step for separating the concentrated salt components provided in step (B) into formate salts and bicarbonate salts. However, there may also be provided a second separating unit specifically configured to carry out the formate separating step. In one embodiment, the at least one separating unit is selected from any of a vacuum or atmospheric still, an evaporator, a rotary evaporator, a cooling bath configured for crystallisation, a membrane separation unit like a reverse osmosis unit, and an electrochemical separation unit like electrodialysis.

The at least one separating unit may be in communication with the reduction reactor. Typically, such a communication is provided by (a) conduit(s) and (a) pump(s). Through the communication with the reduction reactor, water provided in step (B) may be transferred to the reduction reactor. The at least one separating unit may either be in direct communication with the reduction reactor, or be in communication with the reduction reactor over, e.g., a dissolving unit (as further discussed below). The configuration of the communication is not particularly limited. In a preferred embodiment, the communication is configured to transfer water provided in step (B) to the reduction reactor. This configuration may include seals, flanges, pumps, inert conduit linings, etc. The communication may include further intermediary units and/or storage reservoirs. Through the communication with the reduction reactor, the aqueous reaction system comprising formate formed in step (A) may be transferred to the at least one separating unit. Similarly, concentrated salt components comprising formate and bicarbonate formed in step (B) may be transferred to the reduction reactor. This may be done using one communication for both transfers, or multiple communications.

The reduction reactor and the at least one separating unit may also be formed as a single structural unit. That is, a single unit may be provided which is selectively configured as the reduction reactor (as discussed above) and as the at least one separating unit (as discussed above). For example, the single unit may be configured as the reduction reactor to carry out step (A) of the above-described method, and may also be configured as the at least one separating unit to subsequently carry out step (B) of the above-described method. However, the reduction reactor and the at least one separating unit may well also be provided as separate structural units which are in communication with each other (as discussed above).

The hydrogen generating unit is a unit configured to generate hydrogen from water. The presently claimed apparatus may include more than one hydrogen unit. A plurality of hydrogen generating units may be connected in parallel or in sequence. The hydrogen generating unit is not particularly limited and may include any unit customarily used in the art, such as a photosynthesis unit, a thermal decomposition boiler, or an electrolysis cell. In a preferred embodiment, the hydrogen generating unit is an electrolysis cell, a high temperature electrolysis cell, a high-pressure electrolysis cell, a proton-exchange membrane electrolysis cell, an anion exchange electrolysis cell, and a supercritical water electrolysis cell. Combinations of these are possible as well.

The hydrogen generating unit is in communication with the at least one separating unit and with the reduction reactor. Typically, such a communication may be provided by conduits and pumps.

Through the communication connecting the hydrogen generating unit with the at least one separating **unit,** water provided in step (B) may be transferred to the hydrogen generating unit. The configuration of this communication is not particularly limited. In a preferred embodiment, the communication is configured to transfer water provided in step (B) to the hydrogen generating unit. This configuration may include seals, flanges, pumps, inert conduit linings, etc. Water may be transferred from the at least one separating unit to the hydrogen generating unit as liquid water, steam, or as a vapour. The communication may include further intermediary units and/or storage reservoirs.

Through the communication connecting the hydrogen generating unit with the reduction reactor, hydrogen generated in the hydrogen generating unit is transferred to the reduction reactor. The configuration of this communication is not particularly limited. In a preferred embodiment, the communication is configured to transfer hydrogen formed in step (c) to the reduction reactor (i.e., for the reduction reaction performed in step (A)). This configuration may include seals, flanges, compressors, inert conduit linings, etc. The communication may include further intermediary units and/or storage reservoirs, such as pressurized hydrogen tanks.

In a preferred embodiment, separating the concentrated salt components comprising formate provided in step (B) may be carried out using the at least one separating unit discussed above, e.g. by separating water. This also applies to concentrated salt components comprising formate and bicarbonate provided in step (B).

In a preferred embodiment, the apparatus further comprises a concentrated salt component separating unit configured to separate the concentrated salt components comprising formate, and optionally bicarbonate, from the aqueous reaction mixture to provide formate salt, and optionally bicarbonate salt. The present disclosure also includes a method using such a concentrated salt component separating unit to separate the concentrated salt components comprising formate, and optionally bicarbonate, from the aqueous reaction mixture to provide formate salt, and optionally bicarbonate salt. The presently claimed apparatus may include more than one concentrated salt component separating unit. A plurality of concentrated salt component separating units may be connected in parallel or in sequence. Where a plurality of concentrated salt component separating units are present, it is possible that at least one concentrated salt component separating unit is configured to provide formate salt and at least one concentrated salt component separating unit is configured to provide bicarbonate salt. The concentrated salt component separating unit may not be particularly limited. In one embodiment, the concentrated salt component separating unit is selected from any of a vacuum or atmospheric still, an evaporator, a rotary evaporator, a cooling bath configured for crystallisation, a reverse osmosis unit, a filtering unit for solids, and the like. The concentrated salt component separating unit may be in communication with the at least one separating unit. Optionally, the above disclosed concentrated salt component separating units may further include salt separating units configured to separate the formate salt and the bicarbonate from the concentrated salt components comprising formate, and optionally bicarbonate, and optionally, configured to separate formate salts and bicarbonate salts from one another.

In another embodiment, the apparatus may further comprise a dissolving unit configured to dissolve concentrated salt components comprising bicarbonate to provide aqueous bicarbonate. The dissolving unit may also be configured to dissolve concentrated salt components comprising bicarbonate and formate to provide aqueous bicarbonate and formate (i.e., an aqueous reaction system). The presently claimed apparatus may include more than one dissolving unit. A plurality of dissolving units may be connected in parallel or in sequence. The dissolving unit is not particularly limited and may include any unit customarily used in the art. The dissolving unit may be arranged upstream of the reduction reactor. In other words, the dissolving unit may be arranged to be configured to dissolve concentrated salt components comprising bicarbonate (i.e., to perform the dissolving/diluting step described above) prior to step (A) according to the method described above.

The dissolving unit is in communication with the reduction reactor and with the at least one separating unit. Typically, such a communication may be provided by conduits and pumps. The dissolving unit may further be connected to a source or reservoir of bicarbonate salts.

Through the communication connecting the dissolving unit with the at least one separating unit, water provided in step (B) may be transferred to the dissolving unit. The configuration of this communication is not particularly limited. In a preferred embodiment, the communication is configured to transfer water provided in step (B) to the dissolving unit. This configuration may include seals, flanges, pumps inert conduit linings, etc. Water may be transferred from the at least one separating unit to the hydrogen generating unit as liquid water, steam, or as vapour. The communication may include further intermediary units and/or storage reservoirs.

Through the communication between the at least one separating unit and the dissolving unit, concentrated salt components comprising bicarbonate (and optionally formate) formed in step (B) may be transferred to the dissolving unit. In other words, the communication may be further configured to transfer concentrated salt components comprising bicarbonate (and optionally formate) formed in step (B) to the dissolving unit. The bicarbonate recycled for use in step (A) may thus be transferred to the dissolving unit, be dissolved with water by said dissolving unit and then be transferred to the reduction reactor in which step (A) is carried out. The transfer of water and concentrated salt components from the at least one separating unit and the dissolving unit may be done using one communication for both transfers, or multiple communications. For example, one communication may be provided between the at least one separating unit (e.g., a first separating unit) and the dissolving unit for transferring water provided in step (B) to the dissolving unit. Another communication may be provided between the at least one separating unit (e.g., a second separating unit and/or the concentrated salt component separating unit) and the dissolving unit for transferring concentrated salt components comprising bicarbonate (and optionally formate) formed in step (B) to the dissolving unit.

Through the communication connecting the dissolving unit with the reduction reactor, aqueous bicarbonate provided in the dissolving unit is transferred to the reduction reactor. The configuration of this communication is not particularly limited. In a preferred embodiment, the communication is configured to transfer aqueous bicarbonate formed in the dissolving step carried out by the dissolving unit to the reduction reactor (i.e., for the reduction reaction performed in step (A). This configuration may include seals, flanges, pumps, compressors, inert conduit linings, etc. The communication may include further intermediary units and/or storage reservoirs.

The dissolving unit and the reduction reactor may also be formed as a single structural unit. That is, a single unit may be provided which is selectively configured as the dissolving unit (as discussed above) and as the reduction reactor (as discussed above). For example, the single unit may be configured as the dissolving unit for dissolving concentrated salt components comprising bicarbonate to provide aqueous bicarbonate, and may also be configured as the reduction reactor to subsequently carry out step (A) of the above-described method. In particular, the dissolving unit, the reduction reactor, and the at least one separating unit may be formed as a single structural unit. However, the dissolving unit and the reduction reactor may well also be provided as separate structural units which are in communication with each other (as discussed above).

In one embodiment, the apparatus may further comprise a heat exchanger configured to exchange heat between the hydrogen generating unit and the at least one separating unit. In a preferred embodiment, the heat exchanger is configured to transfer (waste) heat generated in the hydrogen generating unit to the at least one separating unit. In the method of the first aspect, heat is exchanged between the step of forming hydrogen for use in step (A) and the step of at least partially separating water from the aqueous reaction system. In a preferred embodiment, (waste) heat generated in the step of forming hydrogen for use in step (A) may be used in step (B) for at least partially separating water from the aqueous reaction system to provide water and concentrated salt components comprising formate.

For example, the at least one separating unit may be configured as a crystallization unit. In other words, the at least one separating unit may be configured to carry out the at least partial separation of water by means of crystallization, preferably by means of evaporation crystallization. Evaporation crystallization is understood in the present context as a separation process in which heat is supplied to a solution, causing a solvent to evaporate. This causes the substance dissolved in the solution to crystallize, i.e., the substance is converted into the crystalline state. In the present case, in the at least one separating unit configured as the crystallization unit, heat may be supplied to the aqueous reaction system, causing the water to evaporate. Thereby, concentrated salt components comprising formate (and optionally bicarbonate) are formed in step (B).

The hydrogen generating unit may, for example, be configured as a high-temperature electrolysis unit (cell). In other words, the hydrogen generating unit may be configured to perform high-temperature electrolysis to form hydrogen for use in step (A).

In a preferred embodiment, (waste) heat generated during high-temperature electrolysis performed in the hydrogen generating unit configured as the high-temperature electrolysis unit (cell) can be transferred to the at least one separating unit configured as the crystallization unit for performing evaporation crystallization to provide water and concentrated salt components. The configuration of the manner of exchanging heat between the hydrogen generating unit and the at least one separating unit is not particularly limited. For example, the heat transfer between the hydrogen generating unit and the at least one separating unit may be performed by the heat exchanger as described above. A heat medium or heat media may be used for transferring heat between the hydrogen generating unit and the at least one separating unit. For example, the heat exchanger may be formed by a piping in which a heat medium is circulated. Typically, this may be performed by a pump.

The method of the first aspect and the apparatus of the second aspect may be described in the following, non-limiting exemplary embodiments with reference to the figures.

In a first exemplary embodiment described in Figure 1, concentrated salt components comprising bicarbonate, such as a bicarbonate salt, are introduced (I) to the reduction reactor A (alpha). Therein aqueous bicarbonate is reduced using hydrogen in accordance with step (A). At this stage, the aqueous reaction system comprising water and formate formed in step (A) is transferred (II) to the at least one separating unit B (beta) through the communication between the reduction reactor and the at least one separating unit referred to in (beta). In the at least one separating unit, water is at least partially separated from the aqueous reaction system in accordance with step (B). Concentrated salt components comprising formate are provided and separated from the aqueous reaction system (V). Water provided in step (B) is transferred to the hydrogen generating unit (III) C (gamma) through the communication between the hydrogen generating unit and the at least one separating unit referred to in (gamma). In the hydrogen generating unit C, hydrogen is formed in accordance with step (c) and (C). This hydrogen is then transferred (IV) from the hydrogen generating unit C to the reduction reactor A through the communication between the hydrogen generating unit and the reduction reactor referred to in (gamma).

### Reference aspect - method of storing hydrogen

In a reference aspect, the present disclosure relates to a method of storing hydrogen using a chemical reaction system, wherein the method comprises:
(A) reducing a hydrogen carrier precursor using hydrogen to form a hydrogen carrier and water,
(B) at least partially separating water from the chemical reaction system to provide water and a composition comprising the hydrogen carrier,
(C) using the water provided in step (B) to form hydrogen for use in step (A).

The description above applies, mutatis mutandis, to the reference aspect. In the context of the first and second aspect described above, bicarbonate (HCO₃⁻) forms a hydrogen carrier precursor and formate (HCOO⁻) forms a hydrogen carrier. In particular, the bicarbonate/formate-based aqueous reaction system described above forms a chemical reaction system. Where applicable, all definitions from the first and second aspects apply. Definitions from the reference aspect also apply to the first and second aspect.

The method of storing hydrogen using a chemical reaction system involves customary and common steps. These are, *inter alia,* providing a reaction vessel, such as a reactor suitable to carry out the method; providing the reagents of the reaction system discussed above, such as the hydrogen carrier precursor, the hydrogen carrier, (optionally) a solvent, water and hydrogen; setting appropriate temperature, pressure and reaction run times; and separating the products.

Step (A), step (B), and step (C) of the method according to the reference aspect substantially correspond to step (A), step (B), and step (C), respectively, as described with respect to the first aspect, in which bicarbonate is used as a hydrogen carrier precursor and formate is used as a hydrogen carrier.

In the reference aspect, the water provided in step (B) may be used to form hydrogen for use in step (A), i.e., in a further instance of step (A). In a preferred embodiment, at least the amount of water formed in step (A) is provided in step (B) and used to form hydrogen for use in step (A). In this embodiment of the reference aspect, the amount of hydrogen formed for use in step (A) is at least as high as the amount of water formed in step (A).

In other embodiments of the reference aspect, the composition comprising the hydrogen carrier provided in step (B) may further comprise the hydrogen carrier precursor. In an embodiment of the reference aspect, the composition comprising the hydrogen carrier and the hydrogen carrier precursor provided in step (B) may be recycled for use in step (A).

In an embodiment of the reference aspect, the method comprises:
(a) carrying out step (A) in a reduction reactor configured to execute the process of step (A),
(b) carrying out step (B) in at least one separating unit configured to execute the process of step (B),
(c) forming the hydrogen for use in step (A) using the water provided in step (B) in a hydrogen generating unit.

The reduction reactor, the at least one separating unit, and the hydrogen generating unit may be further characterized as described above with respect to the second aspect.

In further embodiments of the reference aspect, the following chemical reaction systems may be utilized in a method of storing hydrogen:
- carbon dioxide (CO₂) as hydrogen carrier precursor, and methanol (CH₄O) and/or a hydrocarbon (a non-cyclic hydrocarbon, in particular alkanes (CₙH₂ₙ₊₂), alkenes (CₙH₂ₙ), alkines (CₙH₂ₙ₋₂), or a cyclic hydrocarbon, including the cyclic equivalents of the above non-cyclic hydrocarbons) as hydrogen carrier;
- carbon monoxide (CO) as hydrogen carrier precursor, and a hydrocarbon (a non-cyclic hydrocarbon, in particular alkanes (CₙH₂ₙ₊₂), alkenes (CₙH₂ₙ), alkines (CₙH₂ₙ₋₂), or a cyclic hydrocarbon, including the cyclic equivalents of the above non-cyclic hydrocarbons) as hydrogen carrier;
- a sulphur oxide (SₓO_{y}) or sulphur oxide anion ([SₓO_{y}]⁻) as hydrogen carrier precursor, and hydrogen sulfide (H₂S) as hydrogen carrier;
- silicon oxide (SiOₓ) as hydrogen carrier precursor, and a silicon-hydrogen compound (silane; SiₓH_{y}) as hydrogen carrier;
- a boron oxide (BₓO_{y}) as hydrogen carrier precursor, and boron hydride (BH) as hydrogen carrier;
- a phosphorus oxide (PₓO_{y}) or phosphorus oxide anion ([PₓO_{y}]⁻) as hydrogen carrier precursor, and hydrogen phosphate ([HPₓO_{y}]ⁿ⁻) as hydrogen carrier;
- a nitrogen oxide (NₓO_{y}) or nitrogen oxide anion ([NₓO_{y}]⁻) as hydrogen carrier precursor, and ammonia (NH₃) as hydrogen carrier; or
- a metal oxide (MₓO_{y}, with M being a metal preferably selected from alkaline earth metals) as a hydrogen carrier precursor, and metal hydride (MₓH_{y}, with M being a metal preferably selected from alkaline earth metals) as a hydrogen carrier.
Reactions occurring in the chemical reaction systems are a 'hydrogen-storage' hydrogenation reaction of the hydrogen carrier precursor and a 'hydrogen-release' dehydrogenation of the hydrogen carrier reaction.

The method according to the reference aspect may differ from the method according to the first aspect in that the reaction systems described above may not necessarily be aqueous reaction systems. That is, in the method according to the reference aspect, a solvent (preferably an inert solvent) different from water may be used for dissolving the hydrogen carrier precursor prior to step (A) (or no solvent is used at all). The solvent different from water (if any) may be separated in the at least one separating unit as described above with respect to the first aspect. The solvent once separated may be recycled for dissolving the hydrogen carrier precursor. In particular, the solvent may be transferred to the dissolving unit and/or to the reduction reactor by means of the communication as described above with respect to the first aspect. The composition comprising the hydrogen carrier and the hydrogen carrier precursor provided in step (B) may also be recycled for use in step (A). The composition provided in step (B) may be recycled to the dissolving unit and/or to the reduction reactor by means of the communication as described above with respect to the first aspect. The composition comprising the hydrogen carrier and the hydrogen carrier precursor provided in step (B) may also be separated first, i.e., the hydrogen carrier may be separated from the hydrogen carrier precursor in an additional separation step. Subsequently, only the hydrogen carrier precursor may be recycled for use in step (A).

Through the above disclosed means, the present disclosure provides a water-efficient method of storing hydrogen using a typically water-intensive reaction system. The method according to the reference aspect recovers water for generating hydrogen for storage. In particular, the water formed by the reduction of the hydrogen carrier precursor can be used for generating hydrogen in a stochiometric equivalent manner. This means that the amount of hydrogen thus generated is (substantially) the same as the amount of hydrogen used for reducing the hydrogen carrier precursor in the first instance of step (A). The hydrogen thus generated can be used for reducing the hydrogen carrier precursor in a second instance of step (A), thereby forming water in (substantially) the same amount as during the first instance of step (A). Through this process, the water cycle can be controlled and kept independent of the cycle of the hydrogen carrier and hydrogen carrier precursor. The presently disclosed method of storing hydrogen can thus be dynamically adjusted to the local relative water and energy costs. The hydrogen generation can also be kept independent and free of the need for external water or fossil fuels. This means that the above disclosed method is highly suitable in places where water supplies are low but green energy can readily be provided by means of wind and solar, such as deserts.

## Claims

1. A method of storing hydrogen using a bicarbonate/formate-based aqueous reaction system, wherein the method comprises:
(A) reducing aqueous bicarbonate using hydrogen to form formate and water,
(B) at least partially separating water from the aqueous reaction system to provide water and concentrated salt components comprising formate, and
(C) using the water provided in step (B) to form hydrogen for use in step (A) and/or to dissolve concentrated salt components comprising bicarbonate to provide aqueous bicarbonate for use in step (A).

2. The method according to claim 1, wherein the step (A) is preceded by dissolving concentrated salt components comprising bicarbonate to provide aqueous bicarbonate, preferably wherein the concentrated salt components comprising bicarbonate are a bicarbonate salt.

3. The method according to any of claims 1 or 2, wherein
(i) the aqueous bicarbonate of step (A) is at a concentration of 1-10 M, preferably 4-6 M, and/or
(ii) step (A) is carried out in the presence of a catalyst,
preferably wherein the catalyst is a heterogeneous catalyst,
(iii) step (A) is carried out at a temperature of 20-100 °C and a hydrogen pressure of 1-100 bar.

4. The method according to any of claims 1 to 3, wherein the at least partial separation of water is carried out by at least one of vacuum distillation, atmospheric distillation, vacuum evaporation, evaporation, membrane separation, crystallization, extraction, electrochemical separation or precipitation.

5. The method according to any of claims 1 to 4, wherein the concentrated salt components comprising formate further comprise bicarbonate; preferably wherein the molar ratio of formate to bicarbonate is at least 1:1, more preferably at least 4:1, and even more preferably at least 9:1.

6. The method according to any of claims 1 to 5, wherein the concentrated salt components comprising formate are separated from the aqueous reaction system to provide a formate salt, optionally wherein a further formate salt separation step is carried out.

7. The method according to any of claims 1 to 6, wherein the concentrated salt components comprising formate further comprise bicarbonate and wherein these concentrated salt components comprising formate and bicarbonate are recycled for use in step (A).

8. The method according to any of claims 1 to 7, wherein the forming of hydrogen is carried out by any of photosynthesis, thermal decomposition of water or electrolysis, preferably at least one of direct electrolysis, alkaline electrolysis, high temperature electrolysis, high pressure electrolysis, proton-exchange membrane electrolysis, anion exchange electrolysis, and supercritical water electrolysis.

9. The method according to any of claims 1 to 8, wherein the method comprises:
(a) carrying out step (A) in a reduction reactor configured to execute the process of step (A),
(b) carrying out step (B) in at least one separating unit configured to execute the process of step (B),
(c) forming the hydrogen for use in step (A) using the water provided in step (B) in a hydrogen generating unit, and/or transferring the water provided in step (B) to the reduction reactor.

10. An apparatus configured for storing hydrogen using a bicarbonate/formate-based aqueous reaction system, wherein the apparatus comprises:
(alpha) a reduction reactor configured to reduce aqueous bicarbonate using hydrogen to form formate and water,
(beta) at least one separating unit configured to at least partially separate water from the aqueous reaction system to provide water and concentrated salt components comprising formate, wherein the separating unit is in communication with the reduction reactor, and
(gamma) a hydrogen generating unit in communication with the at least one separating unit and with the reduction reactor.

## Patentansprüche

1. Verfahren zur Speicherung von Wasserstoff unter Verwendung eines wässrigen Bicarbonat-/Formiat-basierten Reaktionssystems, wobei das Verfahren umfasst:
(A) das Reduzieren von wässrigem Bicarbonat unter Verwendung von Wasserstoff, um Formiat und Wasser zu bilden,
(B) das zumindest teilweise Abtrennen von Wasser von dem wässrigen Reaktionssystem, um Wasser und konzentrierte Salzkomponenten, die Formiat umfassen, zu bilden, und
(C) das Verwenden des in Schritt (B) gebildeten Wassers zum Bilden von Wasserstoff zur Verwendung in Schritt (A) und/oder zum Auflösen konzentrierter Salzkomponenten, die Bicarbonat umfassen, um wässriges Bicarbonat zur Verwendung in Schritt (A) zu bilden.

2. Verfahren gemäß Anspruch 1, wobei dem Schritt (A) das Auflösen konzentrierter Salzkomponenten, die Bicarbonat umfassen, vorausgeht, um wässriges Bicarbonat bereitzustellen, wobei die konzentrierten, Salzkomponenten, die Bicarbonat umfassen, bevorzugt ein Bicarbonatsalz sind.

3. Verfahren gemäß einem der Ansprüche 1 oder 2, wobei
(i) das wässrige Bicarbonat aus Schritt (A) eine Konzentration von 1-10 M, bevorzugt 4-6 M, aufweist, und/oder
(ii) Schritt (A) in Gegenwart eines Katalysators durchgeführt wird,
wobei der Katalysator bevorzugt ein heterogener Katalysator ist,
(iii) Schritt (A) bei einer Temperatur von 20-100°C und einem Wasserstoffdruck von 1-100 bar durchgeführt wird.

4. Verfahren gemäß einem der Ansprüche 1 bis 3, wobei das zumindest teilweise Abtrennen von Wasser durch mindestens eines von Vakuumdestillation, atmosphärischer Destillation, Vakuumverdampfen, Verdampfen, Membrantrennung, Kristallisation, Extraktion, elektrochemischem Trennen oder Ausfällen erfolgt.

5. Verfahren gemäß einem der Ansprüche 1 bis 4, wobei die konzentrierten Salzkomponenten, die Formiat umfassen, ferner Bicarbonat umfassen; wobei das Molverhältnis von Formiat zu Bicarbonat bevorzugt mindestens 1:1, mehr bevorzugt mindestens 4:1 und noch mehr bevorzugt mindestens 9:1, beträgt.

6. Verfahren gemäß einem der Ansprüche 1 bis 5, wobei die konzentrierten Salzkomponenten, die Formiat umfassen, von dem wässrigen Reaktionssystem abgetrennt werden, um ein Formiatsalz zu bilden, wobei optional ein weiterer Schritt zur Abtrennung des Formiatsalzes durchgeführt wird.

7. Verfahren gemäß einem der Ansprüche 1 bis 6, wobei die konzentrierten Salzkomponenten, die Formiat umfassen, ferner Bicarbonat umfassen und wobei diese konzentrierten Salzkomponenten, die Formiat und Bicarbonat umfassen, zur Verwendung in Schritt (A) zurückgeführt werden.

8. Verfahren gemäß einem der Ansprüche 1 bis 7, wobei die Bildung von Wasserstoff durch Photosynthese, thermische Zersetzung von Wasser oder Elektrolyse, bevorzugt durch mindestens eines von Direktelektrolyse, alkalischer Elektrolyse, Hochtemperatur-Elektrolyse, Hochdruck-Elektrolyse, Protonenaustauschmembran-Elektrolyse, Anionenaustausch-Elektrolyse und Elektrolyse von überkritischem Wasser, erfolgt.

9. Verfahren gemäß einem der Ansprüche 1 bis 8, wobei das Verfahren umfasst:
(a) das Durchführen von Schritt (A) in einem Reduktionsreaktor, der zur Durchführung des Prozesses von Schritt (A) ausgelegt ist,
(b) das Durchführen von Schritt (B) in mindestens einer Trenneinheit, die zur Durchführung des Prozesses von Schritt (B) ausgelegt ist,
(c) das Bilden des in Schritt (A) zu verwendenden Wasserstoffs unter Verwendung des in Schritt (B) gebildeten Wassers in einer Wasserstofferzeugungseinheit und/oder das Überführen des in Schritt (B) gebildeten Wassers in den Reduktionsreaktor.

10. Vorrichtung, die zur Speicherung von Wasserstoff unter Verwendung eines wässrigen Bicarbonat-/Formiat-basierten Reaktionssystems ausgelegt ist, wobei die Vorrichtung umfasst:
(alpha) einen Reduktionsreaktor, der dazu ausgelegt ist, wässriges Bicarbonat unter Verwendung von Wasserstoff zu Formiat und Wasser zu reduzieren,
(beta) mindestens eine Trenneinheit, die dazu ausgelegt ist, Wasser zumindest teilweise aus dem wässrigen Reaktionssystem abzutrennen, um Wasser und konzentrierte Salzkomponenten, die Formiat umfassen, zu bilden, wobei die Trenneinheit mit dem Reduktionsreaktor in Verbindung steht, und
(gamma) eine Wasserstofferzeugungseinheit, die mit der mindestens einen Trenneinheit und mit dem Reduktionsreaktor in Verbindung steht.

## Revendications

1. Procédé de stockage d'hydrogène utilisant un système réactionnel aqueux à base de bicarbonate/formiate, dans lequel le procédé comprend :
(A) la réduction de bicarbonate aqueux en utilisant de l'hydrogène pour former du formiate et de l'eau,
(B) la séparation d'au moins une partie de l'eau du système réactionnel aqueux pour fournir de l'eau et des composants salins concentrés comprenant un formiate, et
(C) l'utilisation de l'eau fournie à l'étape (B) pour former de l'hydrogène destiné à être utilisé dans l'étape (A) et/ou pour dissoudre des composants salins concentrés comprenant un bicarbonate pour fournir un bicarbonate aqueux destiné à être utilisé dans l'étape (A).

2. Procédé selon la revendication 1, dans lequel l'étape (A) est précédée de la dissolution de composants salins concentrés comprenant un bicarbonate pour fournir un bicarbonate aqueux, de préférence dans lequel les composants salins concentrés comprenant un bicarbonate sont un sel de bicarbonate.

3. Procédé selon l'une quelconque des revendications 1 ou 2, dans lequel
(i) le bicarbonate aqueux de l'étape (A) est à une concentration de 1 à 10 M, de préférence de 4 à 6 M, et/ou
(ii) l'étape (A) est effectuée en présence d'un catalyseur, de préférence, dans lequel le catalyseur est un catalyseur hétérogène,
(iii) l'étape (A) est effectuée à une température de 20 à 100 °C et à une pression d'hydrogène de 1 à 100 bar.

4. Procédé selon l'une quelconque des revendications 1 à 3, dans lequel la séparation d'au moins une partie de l'eau est effectuée par au moins l'une parmi la distillation sous vide, la distillation atmosphérique, l'évaporation sous vide, l'évaporation, la séparation membranaire, la cristallisation, l'extraction, la séparation électrochimique ou la précipitation.

5. Procédé selon l'une quelconque des revendications 1 à 4, dans lequel les composants salins concentrés comprenant un formiate comprennent en outre un bicarbonate ; de préférence dans lequel le rapport molaire formiate sur bicarbonate est d'au moins 1:1, plus préférablement d'au moins 4:1, et encore plus préférablement d'au moins 9:1.

6. Procédé selon l'une quelconque des revendications 1 à 5, dans lequel les composants salins concentrés comprenant un formiate sont séparés du système réactionnel aqueux pour fournir un sel de formiate, facultativement dans lequel une étape supplémentaire de séparation de sel de formiate est effectuée.

7. Procédé selon l'une quelconque des revendications 1 à 6, dans lequel les composants salins concentrés comprenant un formiate comprennent en outre un bicarbonate et dans lequel ces composants salins concentrés comprenant un formiate et un bicarbonate sont recyclés en vue de leur utilisation dans l'étape (A).

8. Procédé selon l'une quelconque des revendications 1 à 7, dans lequel la formation d'hydrogène est effectuée par l'une quelconque parmi la photosynthèse, la décomposition thermique de l'eau ou électrolyse, de préférence au moins l'une parmi l'électrolyse directe, l'électrolyse alcaline, l'électrolyse à haute température, l'électrolyse à haute pression, l'électrolyse à membrane échangeuse de protons, l'électrolyse par échange d'anions et l'électrolyse de l'eau supercritique.

9. Procédé selon l'une quelconque des revendications 1 à 8, dans lequel le procédé comprend :
(a) la réalisation de l'étape (A) dans un réacteur de réduction configuré pour exécuter le procédé de l'étape (A),
(b) la réalisation de l'étape (B) dans au moins une unité de séparation configurée pour exécuter le procédé de l'étape (B),
(c) la formation de l'hydrogène destiné à être utilisé dans l'étape (A) en utilisant l'eau fournie à l'étape (B) dans une unité de génération d'hydrogène et/ou le transfert de l'eau fournie à l'étape (B) vers le réacteur de réduction.

10. Appareil configuré pour stocker de l'hydrogène en utilisant un système réactionnel aqueux à base de bicarbonate/formiate, dans lequel l'appareil comprend :
(alpha) un réacteur de réduction configuré pour réduire un bicarbonate aqueux en utilisant de l'hydrogène pour former un formiate et de l'eau,
(bêta) au moins une unité de séparation configurée pour séparer au moins partiellement l'eau du système réactionnel aqueux pour fournir de l'eau et des composants salins concentrés comprenant un formiate, dans lequel ladite unité de séparation est en communication avec le réacteur de réduction, et
(gamma) une unité de génération d'hydrogène en communication avec l'au moins une unité de séparation et avec le réacteur de réduction.
